# EUROPÄISCHE PATENTANMELDUNG

(11) **EP 2 025 285 A1**
(43) Veröffentlichungstag der Anmeldung: **18.02.2009**
(21) Anmeldenummer: 08160582.6
(22) Anmeldetag: 17.07.2008
(51) Int. Cl.: A61B 5/00, A61B 5/0215

(54) **Implantierbare Druckmesseinrichtung und Anordnung zur Innendruckmessung in einem Blutgefäß**

(30) Priorität: 17.08.2007 DE 102007038801
(71) Anmelder: BIOTRONIK CRM Patent AG, 6341 Baar (CH)
(72) Erfinder: Wildau, Hans-Jürgen, 10717, Berlin (DE); Czygan, Gerald, 91054, Buckenhof (DE); Diebold, Michael, 12169, Berlin (DE); Lippert, Michael, 91522, Ansbach (DE); Harder, Claus, 91080, Uttenreuth (DE); Schmitz-Rode, Thomas, 52070, Aachen (DE)
(74) Vertreter: Lindner-Vogt, Karin L.

(57) **Zusammenfassung**

Implantierbare Druckmesseinrichtung zur Innendruckmessung in einem Blutgefäß oder Herzen eines Patienten, mit einem Drucksensor mit elektrischem Signalausgang, an den vorgesehenen Messort angepassten Fixierungsmitteln zur Fixierung des Drucksensors, einer Stromversorgungseinrichtung des Drucksensors, einer mit dem Signalausgang des Drucksensors leitungsmäßig verbundenen Signalerfassungseinrichtung und einer mit einem Messdatenausgang der Signalerfassungseinrichtung verbundenen Sendeeinrichtung, insbesondere zur drahtlosen Übertragung von Messdaten zu einer Auswertungseinrichtung, insbesondere nach außerhalb des Patientenkörpers.

## Beschreibung

Die Erfindung betrifft eine implantierbare Druckmesseinrichtung zur Innendruckmessung in einem Blutgefäß oder im Herzen und des weiteren eine um eine Auswertungseinheit (ein "Patientengerät") erweiterte Anordnung.

Die kontinuierliche Überwachung des Körperzustandes von Patienten mit chronischen Leiden ist essentiell für die Gewährleistung einer optimalen Therapie. Speziell Patienten mit Herzkrankheiten bedürfen einer Überwachung des Herz- und Kreislaufzustandes, um ihre medikamentöse Therapie zu kontrollieren und zu steuern, akute Verschlechterungen vorhersehen zu können und eine Dekompensation zu verhindern. Für Patienten mit Herzschäden sind diverse Überwachungskonzepte vorgeschlagen worden, u.a. die periodische Erfassung des Körperzustandes in der Klinik, aber auch die Überwachung mit externen Mess- bzw. Überwachungseinrichtungen, etwa zur Kontrolle des Gewichtes, des Blutdrucks, der Zeitabhängigkeit des Pulses etc. In diesem Zusammenhang werden auch seit langem "telemedizinische" Konzepte zur Fernüberwachung des Patienten in seinem normalen Lebensumfeld diskutiert.

In vielen Fällen sind implantierbare Messeinrichtungen gegenüber anderen Verfahren bevorzugt, weil sie die relevanten Größen direkt im Körper bestimmen und daher grundsätzlich genauer und zuverlässiger als externe Verfahren arbeiten können. Zudem können diese Messungen beim heutigen Stand der Technik weitgehend automatisiert und mit Datenfernübertragung ablaufen, ohne dass der Patient selbst oder das ihn überwachende medizinische Personal wesentlich eingreifen müssten.

Bei der Überwachung des hämodynamischen Zustandes von Patienten mit Herzkrankheiten stellt der Blutdruck eine der wichtigsten Größen dar, die überwacht werden sollten. Idealerweise sollte der end-diastolische Druck im linken Ventrikel (LV) oder der Druck im linken Atrium (LA) erfasst werden. Da der Zugang zur linken Seite des Herzens erheblich risikobehaftet ist, hat sich die Überwachung des Blutdrucks in der pulmonalen Arterie (PA) als hinreichend verlässliches Mittel zur Überwachung der Pumpleistung des Linksherzens etabliert. Der PA-Druck korreliert hinreichend mit dem LA-Druck, unter der Annahme, dass der Lungenzustand stabil ist.

Drucksensoren zur Erfassung des Innendruckes in einem Flüssigkeitsgefäß sind in großer Vielzahl bekannt und in der Technik auch im praktischen Einsatz. Viele der in der Technik üblichen Lösungen sind jedoch nicht ohne weiteres auf Messaufgaben im lebenden Organismus übertragbar, weil sie nicht kompakt genug, zu energieaufwendig im Betrieb oder zu anfällig gegenüber Beeinträchtigungen ihrer Funktion durch biologisches Material sind oder in diesem speziellen Einsatzfeld sonstige Unzulänglichkeiten zeigen.

Gleichwohl sind seit Jahren auch Druckmesseinrichtungen für den Einsatz im lebenden Organismus, speziell auch in menschlichen Blutgefäßen, bekannt. Lediglich beispielhaft wird in diesem Zusammenhang hingewiesen auf die neueren Patentveröffentlichungen EP 1117982 A1, US 2002/0045921, US 6,645,143 oder US 6,743,180.

Aufgabe der Erfindung ist die Bereitstellung einer verbesserten Druckmesseinrichtung mit der oben genannten Funktion sowie einer entsprechenden Messanordnung, welche insbesondere über längere Zeit genau und zuverlässig arbeiten, kostengünstig herzustellen und leicht und gefahrlos im Körper eines Patienten zum Einsatz zu bringen sind.

Diese Aufgabe wird durch eine Druckmesseinrichtung mit den Merkmalen des Anspruchs 1 und eine Anordnung mit den Merkmalen des Anspruchs 22 gelöst. Zweckmäßige Fortbildungen des Erfindungsgedankens sind Gegenstand der abhängigen Ansprüche.

Die Erfindung schließt den wesentlichen Gedanken ein, zur Lösung der Aufgabe der Innendruckmessung (in-vivo) in einem Blutgefäß oder ggf. auch dem Herzen einen Drucksensor mit elektrischem Signalausgang und zugehöriger Stromversorgungseinrichtung vorzusehen, dessen Signal im Körper erfasst und dann drahtlos zur weiteren Auswertung/Verwendung nach außerhalb des Körpers übertragen wird. Dem steht in einem angemessen breiten Verständnis der Erfindung indes nicht entgegen, dass die Messdaten auch innerhalb des Körpers - etwa zur Steuerung einer Körperfunktions-Unterstützungseinrichtung - genutzt werden können und in dieser Ausgestaltung keine Übertragung nach außerhalb des Körpers erfolgen muss.

Derzeit wird als zweckmäßig der Einsatz eines kapazitiven Drucksensors angesehen, grundsätzlich kommen aber auch nach anderen physikalischen Wirkprinzipien funktionierende, insbesondere ohne bewegliche Teile auskommende Drucksensoren in Betracht, so etwa piezoelektrische oder piezoresisitive Sensoren.

Ein wesentlicher Akzent der Erfindung liegt im Vorsehen an den vorgesehenen Messort angepasster Fixierungsmittel zur Fixierung des Drucksensors. Als solches Fixierungsmittel kommt ein mit einem Drucksensorkörper verbundenes expandierbares Gefäßwandimplantat (Stent) in Betracht. Derartige Stents sind seit längerem in zahlreichen Ausführungen bekannt und auf dem Markt, so dass ohne weiteres auf kommerziell verfügbare Fixierungsmittel mit geeignetem Aufbau und passender Geometrie zurückgegriffen werden kann. Bei der Auswahl spielt der grundsätzliche Einsatzort - etwa die pulmonale Arterie - eine bestimmende Rolle, es ist aber natürlich auch die individuelle Anatomie des einzelnen Patienten zu berücksichtigen.

Dies gilt selbstverständlich auch für weitere mögliche Ausführungen der Fixierungsmittel, etwa als elastisch abstehende oder abspreizbare Gefäßwandstützen, beispielsweise in Form eines "Dreibeines" aus Silikon. Es gilt in gleichem Maße für eine Ausbildung als Stützspirale oder -helix oder dergleichen Fixierungsmittel, welche sich durch elastisch deformierbare Bogenabschnitte auszeichnen, die eine problemlose Implantation ermöglichen und sich im eingesetzten Zustand der Druckmesseinrichtung an die entsprechende Gefäßwand anlegen sollen.

Erwähnenswert sind auch Fixierungsmittel im weiteren Sinne, welche die Positionierung des Drucksensors in geeigneter Lagebeziehung zur Wandung des Blutgefäßes (oder Herzens) ohne Wandkontakt bewerkstelligen können, etwa eine strömungsgünstige Ausführung des Drucksensorkörpers oder Strömungsleitmittel an diesem, die seine dynamische Positionierung in einem Blutstrom bewirken. In Betracht kommen im übrigen auch ballonartige Fixierungsmittel, welche den Effekt einer dynamischen strömungsgeführten Fixierung mit demjenigen einer wandständigen Fixierung verknüpfen.

Um, speziell mit wandständig wirkenden Fixierungsmitteln, den Drucksensor einerseits während seines Einsatzes in geeigneter Position und stabil im Blutgefäß positionieren und ihn andererseits nach Beendigung der Messaufgabe wieder leicht entfernen zu können, ist bevorzugt eine lösbare Verbindung zwischen dem Drucksensorkörper und den Fixierungsmitteln vorgesehen. Diese sichert, dass der Drucksensor ohne Verletzung der Gefäßwand (an der die Fixierungsmittel dann verbleiben) explantiert werden kann. Alternativ kann auch vorgesehen werden, dass der Drucksensor so mit den Fixierungsmitteln fest verbunden ist, dass er zusammen mit den Fixierungsmitteln verletzungsarm explantiert werden kann, indem die Fixierungsmittel leicht von der Wand, also vom Gewebe, gelöst werden können. Die Fixierungsmittel können als rückziehbare oder ausschraubbare aktive Fixierungsmittel ausgebildet sein oder aber alternativ als vom Drucksensorkörper abstehende passive Fixierungsmittel, die angeklappt werden können, geformt sein.

In weiterer Ausgestaltung der Erfindung ist vorgesehen, dass die Signalerfassungseinrichtung ein separates Gehäuse aufweist, das zur Implantation entfernt vom Drucksensor, außerhalb des Blutgefäßes oder Herzens, ausgebildet ist. Das separate Gehäuse kann etwa im Brustkorb des Patienten angeordnet sein. In weiterer Ausgestaltung nimmt es neben der Signalerfassungseinrichtung die Stromversorgungseinrichtung des Drucksensors auf, und es ist eine Stromversorgungs-Leitungsverbindung zwischen der Stromversorgungseinrichtung und dem Drucksensor vorgesehen. Weiter bevorzugt ist in dem Gehäuse der Signalerfassungseinrichtung zusätzlich eine Steuereinrichtung zur Steuerung der Sensor- und Sendefunktion und optional der Stromversorgung vorgesehen. Es ist schließlich besonders sinnvoll, wenn es sich bei dem Gehäuse um das Gehäuse eines implantierbaren Therapiegerätes, insbesondere Herzstimulationsgerätes, handelt. Speziell wenn die Druckmesseinrichtung im Zusammenhang mit der Funktion eines solchen Therapiegerätes benötigt wird, verringert sich dadurch erheblich der Herstellungs- und Implantationsaufwand.

Eine hierzu alternative Ausführung sieht vor, dass die Stromversorgungseinrichtung, Signalerfassungseinrichtung, Sendeeinrichtung und eine Steuereinrichtung zur Steuerung der Sensor- und Telemetriefunktion und optional der Stromversorgung in einem Drucksensorkörper aufgenommen sind. Es versteht sich, dass dann einer Miniaturisierung der besagten Funktionseinheiten besonderes Augenmerk zu schenken ist, um die in diesem Sinne integrierte Druckmesseinrichtung insgesamt kompakt gestalten zu können.

In einer weiteren Ausführung der Erfindung ist vorgesehen, dass der Signalerfassungseinrichtung eine Vorverarbeitungseinrichtung und ein Messwert-Pufferspeicher zur Messwert-Vorverarbeitung und internen Zwischenspeicherung nachgeschaltet sind. Dies kann sowohl bei der integrierten Ausführung der Messeinrichtung als auch bei der Ausführung sinnvoll sein, in der der eigentliche Drucksensor und die übrigen Komponenten baulich nicht integriert sind. Es trägt speziell zu einer Reduzierung des Datenübertragungsaufwandes zwischen dem Körper des Patienten und einer externen Auswertungseinheit bei, da die Datensammlung und -vorverarbeitung, insbesondere eine Mittelwertbildung über bestimmte Messperioden, intrakorporal erfolgen kann.

In einer weiteren Ausgestaltung der Erfindung ist vorgesehen, dass der Drucksensor und optional die Fixierungsmittel eine endotheliasierungs- bzw. thrombose-hemmende Beschichtung aufweisen. Obgleich sich die vorgeschlagene Messeinrichtung durch eine vergleichsweise große Unempfindlichkeit gegenüber Ablagerungen von biologischem Material auszeichnet, wenn ein Drucksensor ohne bewegte Teile zum Einsatz kommt, vermögen diese letztlich, wenn sie ein bestimmtes Maß übersteigen, auch bei der vorgeschlagenen Einrichtung die Funktion zu beeinträchtigen. Daher ist eine geeignete Beschichtung oder Einkapselung des gesamten Drucksensorkörpers oder jedenfalls der messsignalaufnehmenden Abschnitte zur Verhinderung von Anlagerungen oder zu Verhinderung zur Bildung von Thrombosen von Wert für einen Langzeitbetrieb mit hoher Messgenauigkeit und Zuverlässigkeit beziehungsweise für eine Verhinderung des Ablösens von Thromben.

Unter dem Gesichtspunkt einer effizienten, kostengünstigen Stromversorgung der Druckmesseinrichtung ist bevorzugt, dass die Stromversorgungseinrichtung ein Primär- oder Sekundärelement aufweist. Mit Blick auf die weite Verbreitung von Li-Ionen-Elementen bei Schrittmachern oder anderen implantierbaren Therapiegeräten erscheint die Wahl einer solchen Stromversorgung auch bei der vorgeschlagenen Druckmesseinrichtung als zweckmäßig. In Kombination hiermit, aber auch unabhängig hiervon, kann die Stromversorgung eine Energie-Empfangseinrichtung zur drahtlosen Energiezufuhr von außerhalb des Körpers, insbesondere zum drahtlosen Aufladen eines Sekundärelementes, aufweisen. Es kann sich hierbei etwa um eine induktive Einrichtung handeln, wie sie bei Körperpflegegeräten üblich und kostengünstig kommerziell erhältlich ist.

In einer weiteren Ausgestaltung umfasst die Druckmesseinrichtung mindestens einen zweiten Sensor zur Messung einer zusätzlichen Größe im Körper des Patienten. Hierbei kann als zweiter Sensor ein weiterer Drucksensor in Prandtl-Rohr-Anordnung mit dem ersten Drucksensor zur Gewinnung eines Strömungsgeschwindigkeitssignals vorgesehen sein. Alternativ oder auch in Kombination hiermit kann auch ein Temperatursensor zur Bestimmung der Bluttemperatur und/oder zur Temperatur-Kompensation des Druckmesssignals vorgesehen sein. Durch das Vorsehen zusätzlicher Sensoren lässt sich also einerseits ein Beitrag zur Erweiterung des Messgrößenspektrums und andererseits ein Beitrag zur Erhöhung der Aussagekraft der Ergebnisse der Druckmessung leisten. Mit den hier beispielhaft genannten Varianten sind die Möglichkeiten einer solchen Mehr-SensorEinrichtung hierbei keineswegs ausgeschöpft.

Die im Rahmen der Erfindung vorgeschlagene Gesamtanordnung umfasst neben der Druckmesseinrichtung eine zur extrakorporalen Platzierung ausgebildete Auswertungseinheit, welche eine externe Telemetrieeinheit zur Messdaten- und optional auch Steuersignalverbindung mit der Sendeeinrichtung der Druckmesseinrichtung aufweist. Diese enthält bevorzugt einen atmosphären Drucksensor und eine Druck-Kompensationseinrichtung zur Berechnung eines atmosphärendruck-korrigierten Innendruckwertes. Zusätzlich oder alternativ hierzu kann vorgesehen sein, dass eine mit dem Drucksensor und einem Temperatursensor der implantierten Druckmesseinrichtung verbundene Temperatur-Kompensationseinrichtung zur Berechnung eines bluttemperatur-kompensierten Innendruckwertes vorgesehen ist.

Neben diesen Ausgestaltungen, die die Bereitstellung eines aussagekräftigeren Ergebnisses der Druckmessung zum Ziel haben, kann die Auswertungseinheit auch eine mit dem Drucksensor und/oder weiteren Sensor der implantierbaren Druckmesseinrichtung verbundene Sekundärgrößen-Auswertungseinrichtung aufweisen. Entsprechende Sekundärgrößen können von erheblichem Wert für die Ableitung therapeutischer Maßnahmen aus den Ergebnissen der Innendruckmessung sein. In diesem Sinne kann etwa die Sekundärgrößen-Auswertungseinrichtung zur Bestimmung des Schlagvolumens und/oder der Auswurfleistung des rechten Ventrikels durch Impulsformanalyse des Druckmesssignals der Druckmesseinrichtung ausgebildet sein.

Vorteile und Zweckmäßigkeiten der Erfindung ergeben sich im übrigen aus der nachfolgenden - teils nur skizzenhaften - Beschreibung bevorzugter Ausführungsbeispiele anhand der Figuren. Von diesen zeigen:
- Fig. 1: eine schematische Darstellung einer Ausführungsform der erfindungsgemäßen Messanordnung,
- Fig. 2: eine weitere Darstellung der Messanordnung, in ihrer Anordnung im/zum Körper eines Patienten,
- Fig. 2A: eine Detaildarstellung der Anordnung des Drucksensors in einem Blutgefäß des Patienten und
- Fig. 3: eine Prinzipskizze eines Zwei-Sensoren-Messkopfes.

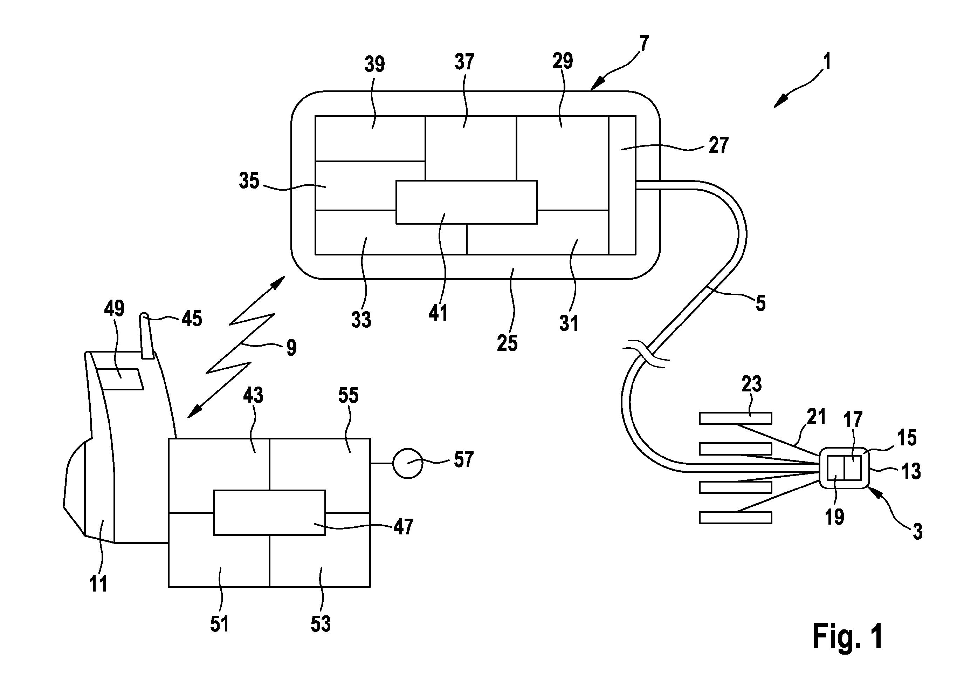

Fig. 1 zeigt skizzenartig eine Messanordnung 1 zur Innendruckmessung in einem Blutgefäß eines Patienten, deren Hauptbestandteile ein Druckmesskopf 3, eine mit diesem über eine Leitung 5 verbundene implantierbare Einheit 7 und eine mit dieser über eine Telemetriestrecke 9 verbundene extrakorporale Auswertungseinheit (Patientengerät) 11 sind.

Der Messkopf 3 umfasst einen Drucksensorkörper 13 mit einer biokompatiblen und thrombose-hemmenden Verkapselung 15, in dem ein kapazitiver Drucksensor 17 und eine Schnittstelleneinrichtung 19 aufgenommen sind. Der Drucksensorkörper 13 ist über Verbindungsmittel 21 lösbar mit einem an die Innenabmessungen der pulmonalen Arterie eines Patienten angepassten Stent 23 verbunden (der hier im bereits expandierten Zustand dargestellt ist).

Die implantierbare Einheit 7 hat ebenso wie der Drucksensorkörper 13 eine biokompatible Hülle 25 und enthält einen Leitungsverbinder 27, eine Hochleistungsbatterie 29 als Stromversorgungseinrichtung des Drucksensors 17, eine Signalerfassungseinrichtung 31, eine Signal-Vorverarbeitungseinrichtung 33, einen Pufferspeicher 35, eine HF-Sende-/Empfangseinrichtung 37 als implantierbare Telemetrieeinheit der Telemetriestrecke 9 und eine zugehörige Antenne 39. Zur Steuerung der Funktionen der implantierbaren Einheit 7 ist schließlich ein Controller 41 vorgesehen.

Das Patientengerät 11 beinhaltet (unter anderem) eine externe HF-Sende-/Empfangseinrichtung 43 mit zugehöriger Antenne 45, einen Controller 47 und eine Bedien- und Anzeigetafel 49. Sie umfasst des weiteren eine eigene Stromversorgung 51 und schließlich eine Druckberechnungsstufe 53, der eine Kompensations-Verarbeitungsstufe 55 zugeordnet ist, die das Signal eines angeschlossenen Atmosphärendruckfühlers 57 zur Luftdruck-Kompensation des Gefäßinnendruck-Messsignals verarbeitet, aber auch weitere Kompensations-Verarbeitungsprozeduren (etwa zur T-Kompensation aufgrund des Signals eines im Messkopf vorgesehenen zusätzlichen T-Sensors) ausführen kann.

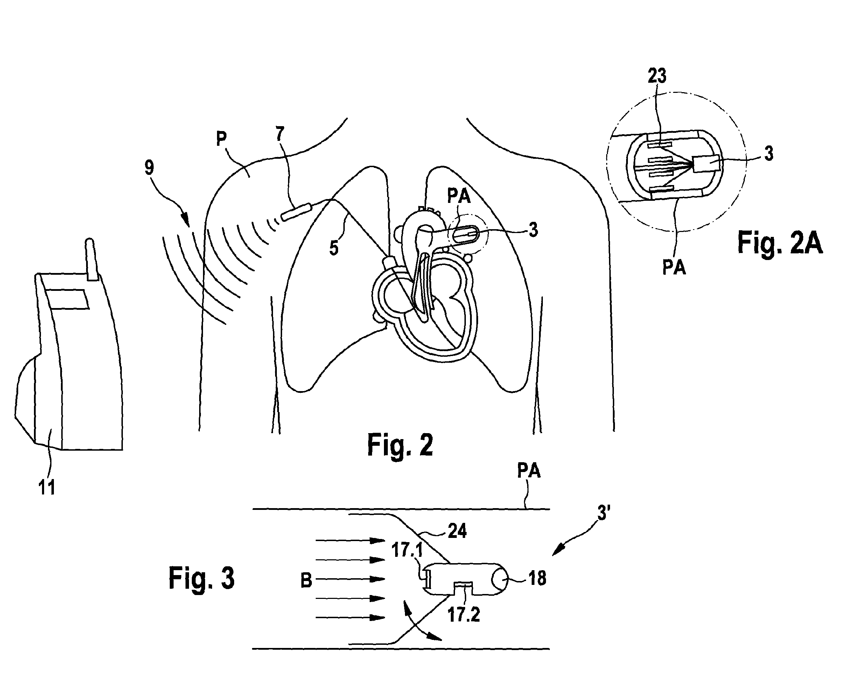

Fig. 2 und 2A zeigen die Anordnung der Komponenten der Messanordnung 1 in Bezug auf den Körper eines Patienten P. Neben der extrakorporalen Platzierung des Patientengerätes 11 ist die Platzierung der implantierbaren Einheit 7 im Brustkorb, unterhalb des Schlüsselbeines, und die Platzierung des Messkopfes 3 in der pulmonalen Arterie PA zu erkennen. Fig. 2A zeigt auch die Abstützung des Messkopfes 3 im Zentrum der pulmonalen Arterie mittels des Stents 23.

In Fig. 3 ist eine modifizierte Ausführung 3' des Messkopfes in dem in die pulmonale Arterie eingesetzten Zustand zu erkennen. Die Pfeile bezeichnen hier die Richtung eines Blutstromes B. Der Messkopf 3' zeichnet sich durch das Vorhandensein zweier Drucksensoren 17.1, 17.2 in räumlicher Anordnung in Art eines Prandtlschen Staurohres zur kombinierten Messung des Druckes und der Strömungsgeschwindigkeit des Blutstroms B aus.

Des Weiteren enthält der Messkopf 3' einen T-Sensor 18 zur gleichzeitigen Erfassung der Bluttemperatur, dessen Signal zur Ermittlung eines genaueren, nämlich T-kompensierten Innendruckwertes genutzt werden kann; vgl. die obige Beschreibung der Messanordnung 1 hinsichtlich des Patientengerätes 11. Schließlich ist in dieser Figur eine modifizierte Ausführung der Fixierungsmittel zur Positionierung des Messkopfes 3' im Zentrum der pulmonalen Arterie PA gezeigt, welche eine Mehrzahl von elastisch andrück- bzw. aufspreizbaren Gefäßwandstützen 24 umfasst. Diese können etwa aus einem Silikon gefertigt und ggf. an eine (hier nicht dargestellte) Silikon-Umhüllung des Messkopfes 3' angeformt sein.

Die Ausführung der Erfindung ist nicht auf die hier erläuterten Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbare Druckmesseinrichtung (3, 7; 3') zur Innendruckmessung in einem Blutgefäß (PA) oder Herzen eines Patienten (P), mit
- einem Drucksensor (17; 17.1, 17.2) mit elektrischem Signalausgang,
- an den vorgesehenen Messort angepassten Fixierungsmitteln (23; 24) zur Fixierung des Drucksensors,
- einer Stromversorgungseinrichtung (29) des Drucksensors,
- einer mit dem Signalausgang des Drucksensors leitungsmäßig verbundenen Signalerfassungseinrichtung (31) und
- einer mit einem Messdatenausgang der Signalerfassungseinrichtung verbundenen Sendeeinrichtung (37), insbesondere zur drahtlosen Übertragung von Messdaten zu einer Auswertungseinrichtung (11), insbesondere nach außerhalb des Patientenkörpers.

2. Druckmesseinrichtung nach Anspruch 1,
wobei die Fixierungsmittel an einem Drucksensorkörper angeordnete Strömungsleitmittel zur blutstrom-induzierten dynamischen Zentrierung des Drucksensors in dem Blutgefäß oder Herzen umfassen oder der Drucksensorkörper zum Bewirken einer blutstrom-induzierten Zentrierung strömungsgünstig gestaltet ist.

3. Druckmesseinrichtung nach Anspruch 1,
wobei die Fixierungsmittel von einem Drucksensorkörper (13) elastisch abstehende oder abspreizbare Gefäßwandstützen (24), eine Stützspirale oder -helix oder dergleichen oder einen Gefäßwandanker zum Einbringen in eine Wandung des Blutgefäßes oder Herzens, insbesondere an einem Verzweigungsabschnitt, aufweisen.

4. Druckmesseinrichtung nach einem der vorangehenden Ansprüche,
wobei die Fixierungsmittel (23, 24) zur Fixierung des Drucksensors in einer pulmonalen Arterie (PA) dimensioniert sind.

5. Druckmesseinrichtung nach einem der vorangehenden Ansprüche,
wobei der Drucksensor (17) mit den Fixierungsmitteln (23) lösbar (21) verbunden ist derart, dass er unter Verbleiben der Fixierungsmittel in dem Blutgefäß aus diesem herausgezogen werden kann.

6. Druckmesseinrichtung nach einem der Ansprüche 1 bis 4, wobei der Drucksensor (17) mit den Fixierungsmitteln (23) fest verbunden ist derart, dass er zusammen mit den Fixierungsmitteln verletzungsarm explantiert werden kann, indem die Fixierungsmittel leicht von der Wand, also vom Gewebe, gelöst werden können.

7. Druckmesseinrichtung nach einem der vorangehenden Ansprüche,
wobei die Signalerfassungseinrichtung (31) ein separates Gehäuse (7) aufweist, das zur Implantation entfernt vom Drucksensor, außerhalb des Blutgefäßes (PA) oder Herzens, ausgebildet ist.

8. Druckmesseinrichtung nach Anspruch 7,
wobei das Gehäuse (7) der Signalerfassungseinrichtung (31) die Stromversorgungseinrichtung (29) des Drucksensors (17; 17.1, 17.2) aufnimmt und eine Stromversorgungs-Leitungsverbindung (5) zwischen der Stromversorgungseinrichtung und dem Drucksensor vorgesehen ist.

9. Druckmesseinrichtung nach einem der Ansprüche 1 bis 6,
wobei die Stromversorgungseinrichtung, Signalerfassungseinrichtung, Sendeeinrichtung und eine Steuereinrichtung zur Steuerung der Sensor- und Telemetriefunktion und optional der Stromversorgung in einem Drucksensorkörper aufgenommen sind.

10. Druckmesseinrichtung nach einem der vorangehenden Ansprüche,
wobei die Stromversorgungseinrichtung (29) ein Primär- oder Sekundärelement aufweist.

11. Druckmesseinrichtung nach einem der vorangehenden Ansprüche,
mit einem zweiten Sensor (17.2) zur Messung einer zusätzlichen Größe im Körper des Patienten.

12. Anordnung (1) zur Innendruckmessung in einem Blutgefäß (PA) oder Herzen eines Patienten (P), mit einer implantierbaren Druckmesseinrichtung (3, 7; 3') nach einem der vorangehenden Ansprüche und einer zur extrakorporalen Platzierung ausgebildeten Auswertungseinheit (11), welche eine externe Telemetrieeinheit (43) zur Messdaten- und optional auch Steuersignalverbindung mit der Sendeeinrichtung (37) der Druckmesseinrichtung aufweist.

13. Anordnung nach Anspruch 12,
wobei die Auswertungseinheit (11) einen atmosphären Drucksensor (57) und eine Druck-Kompensationseinrichtung (55) zur Berechnung eines atmosphärendruck-korrigierten Innendruckwertes aufweist.

14. Anordnung nach Anspruch 12 oder 13,
wobei die Auswertungseinheit (11) eine mit dem Drucksensor (17; 17.1, 17.2) und einem Temperatursensor (18) der implantierten Druckmesseinrichtung (3, 7; 3') verbundene Temperatur-Kompensationseinrichtung (55) zur Berechnung eines bluttemperatur-kompensierten Innendruckwertes aufweist.

15. Anordnung nach einem der Ansprüche 12 bis 14,
wobei die Auswertungseinheit (11) eine mit dem Drucksensor (17; 17.1) und/oder weiteren Sensor (17.2; 18) der implantierbaren Druckmesseinrichtung verbundene Sekundärgrößen-Auswertungseinrichtung aufweist.
